(19)

Europäisches
Patentamt
European
Patent Office
Office européen
des brevets

(11) **EP 2 604 590 A1**

(12) **EUROPEAN PATENT APPLICATION**
published in accordance with Art. 153(4) EPC

(43) Date of publication:
**19.06.2013 Bulletin 2013/25**

(21) Application number: **11816407.8**

(22) Date of filing: **09.08.2011**

(51) Int Cl.:
*C07C 5/333* [(2006.01)]    *B01J 29/86* [(2006.01)]
*C07C 11/08* [(2006.01)]    *C07C 11/09* [(2006.01)]
*C07C 11/167* [(2006.01)]    *C07B 61/00* [(2006.01)]

(86) International application number:
**PCT/JP2011/068098**

(87) International publication number:
**WO 2012/020743 (16.02.2012 Gazette 2012/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.08.2010 JP 2010180953**

(71) Applicant: **Mitsui Chemicals, Inc.**
**Tokyo 105-7117 (JP)**

(72) Inventors:
• **HENG, Phala**
**Sodegaura-shi**
**Chiba 299-0265 (JP)**
• **ICHIKAWA, Shinichiro**
**Sodegaura-shi**
**Chiba 299-0265 (JP)**

• **ISHIKAWA, Junichi**
**Sodegaura-shi**
**Chiba 299-0265 (JP)**
• **IKENAGA, Hirokazu**
**Sodegaura-shi**
**Chiba 299-0265 (JP)**
• **KAWAHARA, Jun**
**Sodegaura-shi**
**Chiba 299-0265 (JP)**
• **GOA, Yasuhide**
**Takaishi-shi**
**Osaka 592-8501 (JP)**

(74) Representative: **Raynor, Stuart Andrew**
**J A Kemp**
**14 South Square**
**Gray's Inn**
**London WC1R 5JJ (GB)**

(54) **METHOD FOR MANUFACTURING UNSATURATED HYDROCARBON, AND DEHYDROGENATION CATALYST USED IN SAID METHOD**

(57)    The invention provides a process for producing an unsaturated hydrocarbon by dehydrogenating a hydrocarbon into a corresponding unsaturated hydrocarbon with use of a nontoxic catalyst having a long catalytic life.

The process for producing unsaturated hydrocarbons includes a step of dehydrogenating a hydrocarbon into a corresponding unsaturated hydrocarbon by contacting the hydrocarbon with a catalyst A that is obtained by supporting zinc and a Group VIIIA metal on a silicate obtained by removing at least part of the boron atoms from a borosilicate.

EP 2 604 590 A1

**Description**

TECHNICAL FIELD

The present invention relates to a process for producing an unsaturated hydrocarbon by contacting a hydrocarbon with a specific dehydrogenation catalyst to dehydrogenate it into a corresponding unsaturated hydrocarbon, and a dehydrogenation catalyst used in the production process.

BACKGROUND ART

Unsaturated hydrocarbons, in particular olefins and dienes, resulting from the dehydrogenation of hydrocarbons are useful basic feedstocks in the petrochemical industry. Exemplary such products are propylene, 1-butene, 2-butene, isobutene, styrene and butadiene.

Propylene is an important industrial material that is used as a feedstock in the synthesis of, for example, acrylonitrile, polypropylene, ethylene/propylene rubber, propylene oxide, acetone, isopropyl alcohol and octanol.

1-Butene and 2-butene are feedstocks used in the metathesis reaction with ethylene to produce propylene. They are also valuable as intermediates for butadiene which has been increasingly demanded as, for example, a feedstock for synthetic rubbers for automobile tires.

Butadiene is a representative diene compound. It is used as a feedstock for synthetic rubbers such as SBR (styrene butadiene rubbers) and NBR (acrylonitrile butadiene rubbers), ABS (acrylonitrile butadiene styrene) resins and nylon 66.

Isobutene is an unsaturated hydrocarbon that is useful as a material for polyisobutylene, methacrolein, methyl methacrylate, methyl tert-butyl ether, ethyl tert-butyl ether, dibutylhydroxytoluene and dibutylhydroxyanisole.

Styrene, which is producible by the dehydrogenation of ethylbenzene, is useful as a feedstock for polystyrene that is a general resin.

Hydrogen that results from the dehydrogenation reaction of hydrocarbons according to the present invention can be directly used as an energy source or as an extremely useful intermediate for the production of petrochemical products.

Processes have been established for the production of these useful unsaturated hydrocarbons. As known in the art, hydrocarbons are dehydrogenated in the presence of a specific catalyst into the corresponding unsaturated hydrocarbons (in particular olefins and/or dienes).

The known dehydrogenation catalysts include chromium oxide-containing catalysts, platinum-tin-containing catalysts, platinum-zinc-containing catalysts and iron-containing catalysts. The chromium oxide-containing catalysts have a very short catalytic life, and chromium is allegedly highly toxic (see, for example, Nonpatent Literatures 1 to 3).

The known platinum-tin-based catalysts for the dehydrogenation processes utilize alumina or $ZnAl_2O_4$ as a support. However, the short catalytic life requires frequent catalyst regeneration, thus complicating the operations (see, for example, Nonpatent Literatures 3 and 4).

Techniques have been reported in which a platinum-zinc-based catalyst is supported on a substantially alkali-free silicalite (see, for example, Patent Literature 1) or on a borosilicate (see, for example, Patent Literatures 2 to 6). The platinum-zinc-based catalysts are described to have a relatively long catalytic life compared to the chromium oxide-containing catalysts and the platinum-tin-containing catalysts.

With regard to the iron-containing catalysts, techniques using iron oxide catalysts containing an alkali metal have been reported (see, for example, Nonpatent Literature 5). However, such catalyst systems have low activity. In particular, severe reaction conditions have to be adopted when the catalysts are used for the reaction of hydrocarbons having low reactivity. Further, a borosilicate that has been deboronated is used as a support of an oxidative dehydrogenation catalyst or an oxidation catalyst (see, for example, Patent Literature 7).

From the industrial viewpoint, a more efficient process is desirable. Namely, it is desired from the energy consumption standpoint that a catalyst be regenerated as few times as possible or require no regeneration. The catalytic life of the known catalysts is insufficient to afford this. A continuous catalyst regeneration facility is provided to remedy the problem that the reaction system has to be suspended each time the catalyst is regenerated. However, the reaction system should be a moving bed system or a fluidized bed system in order that the catalyst to be regenerated can be continuously collected from the reaction system. Designing a process with such a reaction system involves considerable efforts.

There has thus been a need for a process for producing unsaturated hydrocarbons, in particular olefins and dienes, using a catalyst which can catalyze a wide range of hydrocarbons and has high safety and a longer catalytic life to reduce the regeneration frequency.

Citation List

Patent Literatures

Patent Literature 1: JP-A-H02-78439
Patent Literature 2: U.S. Patent No. 4433190
Patent Literature 3: U.S. Patent No. 6197717
Patent Literature 4: U.S. Patent No. 6555724
Patent Literature 5: US2002/0004624A
Patent Literature 6: U.S. Patent No. 5114565
Patent Literature 7: U.S. Patent No. 5324702

Nonpatent Literatures

Nonpatent Literature 1: Catal. Today, 1999, 51, 223.
Nonpatent Literature 2: Canadian Chemical News, 1984, 10, 1924.
Nonpatent Literature 3: Kinetics and Catalysis, 2001, 42, 72.
Nonpatent Literature 4: Petrochemie, 1995, 111, 171.
Nonpatent Literature 5: Handbook of Heterogeneous Catalysis, Vol. 5, 2151, 1997 (edited by G. Ertl et al., VCH, 1997).

SUMMARY OF INVENTION

Technical Problem

It is an object of the invention to provide a process for producing an unsaturated hydrocarbon by dehydrogenating a hydrocarbon into a corresponding unsaturated hydrocarbon with use of a nontoxic catalyst having a long catalytic life, and a catalyst that can be suitably used in the production process.

Solution to Problem

The present inventors carried out studies in order to achieve the above object. They have then developed a catalyst in which zinc and a Group VIIIA metal are supported on a silicate obtained by removing at least part of the boron atoms from a borosilicate. The catalyst has been found to have a markedly long catalytic life compared to the known dehydrogenation catalysts and to afford unsaturated hydrocarbons with a conversion and a selectivity which are substantially equal to those of the conventional catalysts. The invention has been completed based on the findings.

An aspect of the invention is directed to a process for producing unsaturated hydrocarbons which comprises a step of dehydrogenating a hydrocarbon into a corresponding unsaturated hydrocarbon by contacting the hydrocarbon with a catalyst A that is obtained by supporting zinc and a Group VIIIA metal on a silicate obtained by removing at least part of the boron atoms from a borosilicate.

In the catalyst A, the residual rate of the boron atoms in the silicate is preferably not more than 80% relative to all the boron atoms in the borosilicate. The amount of the zinc in the catalyst A is preferably 0.01 to 15% by mass. The amount of the Group VIIIA metal in the catalyst A is preferably 0.01 to 5% by mass. The borosilicate is preferably a MFI zeolite.

Examples of the Group VIIIA metals include palladium, nickel, platinum, rhodium, ruthenium and iridium. Of these, platinum is preferably used.

Examples of the hydrocarbons as the materials in the production process according to the invention include hydrocarbons of 2 to 20 carbon atoms. Propane, n-butane, isobutane, n-butene, isopentane and ethylbenzene are particularly preferable.

Preferably, the unsaturated hydrocarbon produced by the process of the invention is an olefin and/or a diene. Olefins and dienes are valuable industrial materials.

In the production process of the invention, the contacting of the hydrocarbon and the catalyst A is preferably performed at a reaction temperature of 300 to 800°C, and the reaction pressure is preferably 0.01 to 1 MPa.

The contacting of the hydrocarbon and the catalyst A is preferably performed in the presence of water, and the usage amount of water is preferably 0.05 to 20 molar times relative to the hydrocarbon.

Another aspect of the invention is directed to the catalyst A that is used in the inventive process for producing unsaturated hydrocarbons. The catalyst A is obtained by supporting zinc and a Group VIIIA metal on a silicate obtained by removing at least part of the boron atoms from a borosilicate, and can catalyze the dehydrogenation of a hydrocarbon into a corresponding unsaturated hydrocarbon.

Advantageous Effects of Invention

According to the process for producing unsaturated hydrocarbons of the present invention, a hydrocarbon is dehydrogenated into a corresponding unsaturated hydrocarbon with a catalyst which has a long catalytic life to realize infrequent catalyst regeneration and excellent operation properties. The catalyst of the invention can be suitably used in the process.

BRIEF DESCRIPTION OF THE DRAWINGS

FIG. 1 is a diagram that visually illustrates the difference of the catalytic life of catalysts used in Examples 1 and 6 and Comparative Examples 1 and 2.
FIG. 2 is a diagram that visually illustrates the difference of the catalytic life of catalysts used in Example 2 (water was absent from the reaction system), Example 3 (water was present in the reaction system) and Comparative Example 3 (water was present in the reaction system).

DESCRIPTION OF EMBODIMENTS

The catalysts A, the hydrocarbons as production materials, and the dehydrogenation reaction in the present invention will be sequentially described hereinbelow.

[Catalysts A]

A catalyst A that is used in the inventive process for producing unsaturated hydrocarbons (hereinafter, also referred to as the "production process (of the invention)") is obtained by supporting zinc and a Group VIIIA metal on a silicate obtained by removing at least part of the boron atoms from a borosilicate.

(Borosilicates)

The borosilicates used to obtain the catalyst A are not particularly limited as long as they are silicates containing a boron atom. The borosilicates may have a crystalline or amorphous structure. From the viewpoints of the catalytic reaction efficiency and the catalytic life, crystalline borosilicates are preferable.
The crystalline borosilicates have a zeolite structure. Examples of the zeolite structures include MFI, BEA, MWW, CON and FAU. Of these, MFI zeolite (borosilicate) is preferable because of easy availability.
The aluminum content in the crystalline borosilicate (zeolite) used in the invention is not particularly limited. However, the silica/alumina ratio in the borosilicate is preferably not less than 100, more preferably not less than 500, particularly preferably not less than 1000, and most preferably not less than 2000. The ratio is usually not more than 400000, which is the substantial limit of analytical accuracy.
An excessively high aluminum content (an excessively small silica/alumina ratio) in the borosilicate may induce the oligomerization of the unsaturated hydrocarbon produced in the inventive process, and the oligomer can accumulate as coke on the catalyst A to shorten the catalytic life. The advantageous effects of the invention are fully achieved when the silica/alumina ratio is 400000 or below.
The content of alkali metals and alkaline earth metals in the crystalline borosilicate (zeolite) used in the invention is not particularly limited. However, the presence of a large amount of alkali such as potassium in the catalyst A can adversely affect the dehydrogenation reaction. Thus, it is desirable that the borosilicate be substantially free of alkali metals and alkaline earth metals. By the term "substantially free of" is meant that the concentration of alkali metals and that of alkaline earth metals in the borosilicate are each not more than 300 ppm.
The content of the boron atoms in the borosilicate prior to the removal of at least part of the boron atoms is not particularly limited, but is preferably 100 to 30000 ppm, more preferably 500 to 10000 ppm, and particularly preferably 1000 to 8000 ppm. As will be described later, the long life of the catalyst A is probably contributed to by the lattice defects that are formed by the removal of boron atoms. Thus, it is necessary that the borosilicate before the boron atom removal contain a certain amount of boron atoms.
The content of the boron atoms may be measured by, for example, an analytical method using ICP (inductively coupled plasma) (ICP-AES).
The borosilicates may be easily produced by known methods or may be purchased from catalyst manufacturers.
In the preparation of the catalyst A, the borosilicates may be used singly, or two or more may be used in combination.

(Removal of boron atoms from borosilicate)

In the invention, at least part of the boron atoms is removed from the borosilicate. The resultant silicate having a reduced content of boron atoms is used as a support of zinc and a Group VIIIA metal described later.

By removing at least part of the boron atoms from the borosilicate, lattice defects such as atomic vacancies are formed in the resultant silicate (in which a certain amount of boron atoms usually remain). It is considered that the lattice defects greatly contribute to the improvement in dispersibility of zinc and a Group VIIIA metal in the catalyst A, leading to a longer catalytic life. In detail, there is probably some interaction between the lattice defects and the zinc and the Group VIIIA metal. For example, it is probable that the zinc and the Group VIIIA metal as heteroatoms attach stably in the lattice defects of the silicate support, and consequently the agglomeration of metals that is one of the causes for catalyst deactivation is suppressed.

After at least part of the boron atoms are removed from the borosilicate, the residual rate of the boron atoms in the silicate is preferably not more than 80%, more preferably not more than 50%, particularly preferably not more than 30%, and most preferably not more than 20% relative to all the boron atoms (100% by weight) contained in the borosilicate before the boron atom removal. Such a residual rate is preferred from the viewpoint of improving the catalytic life.

The residual rate of the boron atoms may be calculated by comparing the content of the boron atoms in the borosilicate before the boron atom removal, with the content of the boron atoms in the silicate after the boron atom removal. The content of the boron atoms may be measured by, for example, an analytical method using ICP.

To remove at least part of the boron atoms from the borosilicate, methods may be adopted such as treating the borosilicate with an aqueous inorganic or organic acid solution, and treating the borosilicate with a solution of an inorganic or organic acid in an organic solvent (other than water). Of these methods, a treatment with an aqueous inorganic or organic acid solution is preferable in terms of safety and production costs.

Examples of the inorganic acids include nitric acid, sulfuric acid and hydrochloric acid. Examples of the organic acids include acetic acid and oxalic acid. Examples of the organic solvents include methanol and ethanol.

The treatment of the borosilicate with the solution such as the acidic aqueous solution is usually performed at room temperature (25°C) to 200°C. To increase the efficiency in removing the boron atoms, the treatment is preferably carried out at a high temperature. Although raising the treatment temperature is effective to increase the efficiency in the boron atom removal, a treatment temperature of 100 °C or above involves the use of an autoclave, which is complicated to operate. Thus, the treatment is preferably performed at less than 100°C.

In detail, the treatment indicates that the borosilicate is soaked in the solution such as the acidic aqueous solution. From the viewpoint of the efficiency in removing the boron atoms, the concentration of the solution such as the acidic aqueous solution is preferably 0.01 to 15 N (eq/L). The solution such as the acidic aqueous solution may be a single acidic solution or a mixture of two or more acidic solutions.

The treatment time is variable in accordance with the treatment temperature, the amount of the boron atoms in the borosilicate, the removal rate and other factors. Usually, the treatment time is 1 to 24 hours. In some cases, the borosilicate may be repeatedly treated with the solution such as the acidic aqueous solution.

The removal of the boron atoms may be followed by filtration and calcination of the residue to obtain the silicate as powder that is easy to handle. The calcination may be carried out in one stage or two or more stages.

The calcination in one stage may be performed at a heating temperature of 400 to 600°C for 1 to 10 hours.

When the calcination is carried out in two stages, the first stage is performed at a heating temperature of 80 to 150°C for 0.5 to 5 hours and the second stage is performed at a heating temperature of 400 to 600°C for 1 to 10 hours.

(Zinc, Group VIIIA metals, and supporting of these metals)

Zinc and a Group VIIIA metal are supported on the silicate that is obtained by removing at least part of the boron atoms from the borosilicate as described above, resulting in the catalyst A used in the production process of the invention.

The "Group VIIIA metal" is an expression according to the old IUPAC system. In the current IUPAC system, the expression is "Group 8-10 metal". Examples of the Group VIIIA metals include platinum, palladium, ruthenium, iridium, rhodium and nickel. Of these, the metal supported on the silicate is preferably platinum in terms of catalytic activity.

In the supporting on the silicate, the zinc and the Group VIIIA metal may be used as corresponding metal compounds such as metal nitrates, metal chlorides and metal complexes. They may be used to support zinc and the Group VIIIA metal on the silicate by a known method such as an ion exchange method or an impregnation method.

Exemplary zinc compounds include zinc nitrate, zinc chloride and zinc acetate. Exemplary Group VIIIA metal compounds include chloroplatinic acid, tetraammine platinum chloride, tetraammine platinum hydroxide, tetraammine platinum nitrate and tetraammine platinum tetrachloroplatinic acid.

After the zinc and the Group VIIIA metal are supported on the silicate by a method such as an ion exchange method or an impregnation method, the product is calcined. The calcination may be carried out in one stage or two or more stages.

When the calcination is conducted in one stage, the silicate supporting the zinc and the Group VIIIA metal may be heated at 400 to 600°C for 1 to 10 hours.

When the calcination is carried out in two stages, the silicate may be heated in the first stage at 80 to 150°C for 0.5 to 5 hours and in the second stage at 400 to 600°C for 1 to 10 hours.

The calcination atmosphere is not particularly limited as long as it does not contain a reducing gas. In a usual embodiment, the calcination is performed under a stream of air.

The metals may be supported on the silicate in any order without limitation. In an embodiment, the zinc compound may be used first to support the zinc on the silicate, and thereafter the Group VIIIA metal compound may be used to support the metal on the silicate. Alternatively, the Group VIIIA metal compound may be used first to support the metal on the silicate, and thereafter the zinc compound may be used to support the zinc on the silicate. Still alternatively, the zinc compound and the Group VIIIA metal compound may be used simultaneously to support the zinc and the Group VIIIA metal on the silicate at the same time.

The catalyst A for use in the inventive process is obtained by the supporting described above. From the viewpoints of the catalytic life and the catalytic efficiency, the amount of the supported zinc is preferably 0.01 to 15% by mass, more preferably 0.05 to 5% by mass, and particularly preferably 0.1 to 3% by mass relative to the mass of the catalyst (A) (100% by mass).

From the viewpoints of the catalytic life and the catalytic efficiency, the amount of the supported Group VIIIA metal is preferably 0.01 to 5% by mass, more preferably 0.05 to 3% by mass, and particularly preferably 0.1 to 1.5% by mass relative to the mass of the catalyst (A) (100% by mass).

The metal molar ratio of the zinc to the Group VIIIA metal (Zn/Group VIIIA metal) is preferably not less than 0.5. If the ratio is less than 0.5, the catalytic life is short. If the ratio is excessively high, the activity is lowered and byproducts increase. The ratio is usually 0.5 to 50, preferably 1 to 30, and more preferably 1 to 20.

The supported amount in the catalyst A indicates the proportion of the weight in terms of the metal atoms of the metal compound used in the supporting, relative to the total weight of the catalyst A. For example, the supported amounts of the metals (zinc and Group VIIIA metal) in the catalyst A may be directly determined by an analytical method using ICP.

In the present invention, two kinds of the metals, i.e., zinc and Group VIIIA metal, are supported on the silicate that is obtained by removing at least part of the boron atoms from the borosilicate. Needless to mention, a plurality of the Group VIIIA metals may be used (supported).

The catalyst A is loaded into a reactor in the following manner. The catalyst A obtained by the aforementioned method is essentially fine powder. The fine powdery catalyst may be directly loaded into a reactor. To prevent an increase of pressure loss, the catalyst may be loaded as a mixture or may be shaped as described below. In an embodiment, the powdery catalyst may be physically mixed with an inert filler such as silica balls or alumina balls, and the mixture may be loaded into a reactor. In another embodiment, the fine powdery catalyst may be kneaded together with a sintering agent (a binder) that does not alter the catalytic performance, and the kneaded product may be shaped. Silica-based sintering agents may be typically used. Further, the sintering agents may be selected from alumina-based agents, titania-based agents, zirconia-based agents and diatomaceous earth-based agents.

The sintering is preferably performed at a temperature in the range of 500 to 800°C. Exemplary catalyst shapes are tablets, extrudates, pellets, spheres, microspheres, CDS extrudates, trilobes, quadlobes, rings, two-spoke rings, special spoke rings such as HGS, EW and LDP, rib rings and granules.

In the shaping step, the binder content is controlled and a number of shaping auxiliaries such as thickening agents, surfactants, humectants, plasticizers and binder materials are used to ensure that the catalyst A can be shaped without deteriorations of the properties and the catalytic performance of the shaped catalyst A. In a preferred embodiment, the shaping step is performed at an appropriate stage during the catalyst production steps with consideration of the reactivity of these substances. For example, the steps may be carried out in the sequence such that the borosilicate powder is shaped, boron atoms are then removed and thereafter the metals are supported, in the sequence such that boron atoms are removed from the borosilicate, the silicate is then shaped and thereafter the metals are supported, in the sequence such that boron atoms are removed from the borosilicate, the metals are then supported and thereafter the catalyst is shaped, or in the sequence such that boron atoms are removed from the borosilicate, zinc is then supported, the silicate is shaped and thereafter the Group VIIIA metal is supported.

[Hydrocarbons]

In the process for producing unsaturated hydrocarbons according to the invention, a hydrocarbon that corresponds to the target unsaturated hydrocarbon is used as a material. That is, the hydrocarbon used in the process is one that is given by hypothetically hydrogenating the double bond in the target unsaturated hydrocarbon. The hydrocarbons used in the invention usually have 2 to 20 carbon atoms.

The skeleton structures of the hydrocarbons are not particularly limited, and any linear hydrocarbons and branched hydrocarbons may be used. Examples of the linear hydrocarbons include ethane, propane, n-butane, n-butene, n-pentane, n-pentene, n-hexane, n-hexene, n-heptane, n-heptene, ethylbenzene and cumene. Examples of the branched hydrocarbons include isobutane, isobutene, isopentane, isopentene, 2-methylpentane, 2-methylpentene, 3-methylpentane and 2,2-dimethylbutane.

Of the above hydrocarbons, propane, n-butane, n-butene, isobutane, isopentane and ethylbenzene are particularly preferable because the dehydrogenation of these hydrocarbons gives industrially useful unsaturated hydrocarbons having a double bond. Main dehydrogenation products are propylene from propane, 1-butene, 2-butene and isobutene from n-butane, butadiene from n-butene, isobutene from isobutane, isoprene from isopentane, and styrene from ethylbenzene.

Examples of the materials that have the above components include a fraction containing butane that results from the separation of butadiene and butene from a C4 fraction withdrawn from a naphtha thermal cracking furnace or a naphtha catalytic cracking furnace; a fraction containing butane and butene that results from the separation of butadiene from a C4 fraction; a fraction containing pentane that results from the separation of isoprene and pentene from a C5 fraction; and a fraction containing pentane and pentene that results from the separation of isoprene from a C5 fraction. Fractions that are returned to a naphtha cracking furnace and reused as materials, or fractions that are used as fuels may be used as the hydrocarbon materials. Further, LPG (liquefied petroleum gas) that is easily available as fuel may be used as the hydrocarbon material.

The above-described materials may be used singly or may be mixed in appropriate amounts. The materials are not limited to those described above, and may contain other components (impurities) as long as they have $C_{2-20}$ hydrocarbons and the presence of impurities does not destroy the advantageous effects of the invention. Examples of such other components include hydrogen, carbon monoxide, carbon dioxide, methane and dienes.

When the material used in the invention contains a plurality of $C_{2-20}$ hydrocarbons, part of the hydrocarbons may be separated by a known separation method and used in the reaction, or the material may be used as it is without separation and the dehydrogenation product such as olefin or diene may be separated and purified after the dehydrogenation. For example, in the case where LPG which is a mixture of propane, n-butane and isobutane is used as the material, propane, n-butane or isobutane may be separated by distillation purification and used as the material, or the gas may be used directly without separation and the dehydrogenation product such as olefin or diene may be separated after the dehydrogenation.

[Dehydrogenation reaction]

The hydrocarbon described above is brought into contact with the catalyst A. The contact induces a dehydrogenation reaction of the hydrocarbon which forms a double bond between the dehydrogenated carbon atoms, resulting in an unsaturated hydrocarbon corresponding to the hydrocarbon.

Since the hydrocarbon is a reducing substance, the contact of the hydrocarbon with the catalyst A results in reduction of at least part of the metals (zinc and Group VIIIA metal) in the catalyst A. The reduced zinc and Group VIIIA metal form an alloy which is considered to have high catalytic performance in the dehydrogenation reaction.

Thus, the catalyst A may be preliminarily reduced by a pretreatment with a reducing gas such as hydrogen or carbon monoxide before used in the dehydrogenation of the hydrocarbon. The reducing gas may be used without dilution or may be appropriately diluted with a diluent such as water or nitrogen.

The catalyst A may catalyze the hydrocarbon dehydrogenation without such a pretreatment as described above. However, carrying out a pretreatment with a reducing gas such as hydrogen or carbon monoxide is effective in order to shorten the induction period (in which there are very few metals that have been reduced and activated, i.e., the activity of the catalyst A is still low) at an initial stage of the reaction.

From the viewpoint of the reaction efficiency, the temperature for the contact of the hydrocarbon and the catalyst A, namely the dehydrogenation temperature, is preferably 300 to 800°C, more preferably 400 to 700°C, particularly preferably 450 to 650°C, and most preferably 480 to 620°C. Since the production process of the invention is carried out at this relatively high temperature, the dehydrogenation reaction takes place in a gas phase.

If the reaction temperature is low, the equilibrium conversion of the hydrocarbon is decreased and the yield of the unsaturated hydrocarbon in one path tends to be lowered. An excessively high reaction temperature increases the coking rate and thus tends to result in a short life of the catalyst A.

From the viewpoint of the reaction efficiency, the reaction pressure in the dehydrogenation is preferably 0.01 to 1 MPa, more preferably 0.05 to 0.8 MPa, and particularly preferably 0.1 to 0.5 MPa.

Because the production process of the invention is usually carried out in a gas phase, the dehydrogenation reaction is preferably performed in a continuous reactor. In such a case, it is simple and appropriate to express the usage amount of the catalyst A with the weight hourly space velocity (WHSV). Here, the feed rate of the hydrocarbon relative to the catalyst A is not particularly limited. However, the feed rate is preferably such that WHSV (the feed amount of the hydrocarbon per unit amount of the catalyst A and per unit time) is in the range of 0.01 to 50 $h^{-1}$, and more preferably 0.1 to 20 $h^{-1}$.

Further preferred usage amounts of the hydrocarbon and the catalyst A may be determined appropriately in accordance with the dehydrogenation temperature or the activity of the catalyst A. The reaction in the invention may be carried out with such amounts.

In the process for producing unsaturated hydrocarbons according to the invention, the reaction may be carried out in the presence of components other than the hydrocarbon and the catalyst A while still achieving the object and the advantageous effects of the invention. Preferred examples of such components include water, methane, hydrogen and oxides such as carbon dioxides, with water being particularly preferable.

The inventors have found that the presence of water in the dehydrogenation reaction drastically improves the catalytic life. This is probably because water is effective in lowering the rate of coking on the catalyst A and in suppressing the volatilization of zinc from the catalyst A.

The amount of water that is present in the dehydrogenation reaction is preferably 0.05 to 20 molar times, more preferably 0.3 to 10 molar times, and particularly preferably 0.5 to 5 molar times relative to the hydrocarbon as the material (water/hydrocarbon). If water is present in an excessively scarce amount, the effects such as suppressed rate of coking on the catalyst A are not achieved, possibly resulting in a short catalytic life. Adding water in an excessively large amount decreases the heat efficiency, and the energy-based reaction efficiency may be lowered.

Methane can be used as a reaction diluent to produce the same effect as if the pressure in the reaction system is reduced, and consequently the equilibrium conversion can be improved at times. Hydrogen and carbon dioxide can extend the life of the catalyst A at times.

The reactor type used in the process for producing unsaturated hydrocarbons according to the invention is not particularly limited and may be a known type. For example, reactor types such as fixed bed, moving bed and fluidized bed may be adopted. A fixed bed reaction system is particularly preferable because of easy process design.

From the viewpoint of industrial usefulness, the unsaturated hydrocarbons manufactured by the process of the invention are preferably olefins (unsaturated hydrocarbons having a single double bond in the molecule) and dienes (unsaturated hydrocarbons having two double bonds in the molecule). That is, the production process of the invention is preferably a process for producing olefins and/or dienes.

Particularly preferred compounds that are manufactured by the process of the invention are propylene, 1-butene, 2-butene, isobutene, butadiene, isoprene and styrene produced from propane, n-butane, isobutane, n-butene, isopentane and ethylbenzene. Butadiene and isoprene may be manufactured simultaneously from butane and isopentane, and such production of dienes is also within the scope of the production process of the invention. As described hereinabove, these compounds are useful materials in the industry.

As discussed above, the catalyst A used in the production process of the invention has a longer catalytic life than known dehydrogenation catalysts. The catalytic life can be further drastically extended by allowing water to be present in the reaction system. The process of the invention involves the catalyst which requires infrequent catalyst regeneration and has excellent operation properties. Thus, the process of the invention realizes production of olefins and/or dienes at the industrial level.

[Purification step]

In performing the production process of the invention, the products, preferably olefins and/or dienes, obtained at the reactor exit often contain hydrogen, the starting materials, the byproducts, water and the components described hereinabove. That is, the products often contain hydrogen formed in the reaction, paraffins (such as ethane and propane) and olefins (such as ethylene and propylene) that have come to have a shorter carbon chain after the reaction, unreacted hydrocarbons, and impurities contained in the catalyst A or the hydrocarbon materials. The products may be purified by known methods.

EXAMPLES

The present invention will be described in detail by presenting examples hereinbelow without limiting the scope of the invention.

(Synthetic Example 1) Preparation of partially deboronated (i.e., part of boron atoms being removed) silicate (zeolite)

A glass flask equipped with a condenser tube was charged with 9.23 g of MFI borosilicate containing 3200 ppm of boron atoms which had been prepared by the method described later in Synthetic Example 11. Thereafter, 900 ml of a 3 N aqueous nitric acid solution was added to the flask, and the temperature was raised to 100°C while performing stirring. After the water reflux started, the reaction was performed for 18 hours. Thereafter, the slurry liquid was cooled and filtered through a membrane filter (0.5 μm). The residue was washed with 300 ml of distilled water.

The residue was subjected to another cycle of the above treatments (the addition of the aqueous nitric acid solution, the reaction under water reflux, the cooling, the filtration, and the washing of the residue with distilled water). The residue was calcined in air at 120°C for 4 hours and at 540°C for 6 hours. Thus, 8.7 g of partially deboronated silicate (zeolite) was obtained.

The amount of the boron atoms in the silicate powder was determined to be 260 ppm by ICP-AES (residual rate of the boron atoms: approximately 8%).

(Synthetic Example 2) Preparation of zinc-supported silicate (zeolite)

An aqueous solution weighing 0.33 g that contained 0.0810 g (0.272 mmol) of zinc nitrate hexahydrate was added to 1 g of the silicate obtained in Synthetic Example 1. The zinc ions were allowed to impregnate the silicate by the incipient wetness method.

After impregnated with the solution, the powder was sufficiently agitated and was calcined in air at 120°C for 3 hours and at 500°C for 4 hours. Thus, zinc-supported silicate was prepared.

(Synthetic Example 3) Preparation of platinum- and zinc-supported silicate (catalyst 1)

An aqueous solution weighing 0.33 g that contained 0.00846 g (0.0164 mmol) of chloroplatinic acid hexahydrate was added to 1 g of the zinc-supported silicate obtained in Synthetic Example 2. The platinum ions were allowed to impregnate the silicate by the incipient wetness method.

After impregnated with the solution, the powder was sufficiently agitated and was calcined in air at 120°C for 3 hours and at 500°C for 4 hours. Thus, platinum- and zinc-supported silicate (catalyst 1) was prepared.

The (supported) amount of platinum and the (supported) amount of zinc in the catalyst 1 were determined by ICP-AES to be 0.32% by mass and 1.78% by mass, respectively.

(Synthetic Example 4) Preparation of platinum- and zinc-supported borosilicate (catalyst 2)

Zinc-supported borosilicate was prepared in the same manner as in Synthetic Example 2, except that the partially deboronated silicate (zeolite) was replaced by borosilicate without the boron removal, namely, MFI borosilicate containing 3200 ppm of boron atoms which had been prepared by the method described later in Synthetic Example 11.

Platinum- and zinc-supported borosilicate (catalyst 2) was prepared in the same manner as in Synthetic Example 3, except that the zinc-supported silicate obtained in Synthetic Example 2 was replaced by the zinc-supported borosilicate obtained above.

The (supported) amount of platinum and the (supported) amount of zinc in the borosilicate catalyst 2 were determined by ICP-AES to be 0.32% by mass and 1.78% by mass, respectively.

(Synthetic Example 5) Preparation of zinc-supported silicate (zeolite)

An aqueous solution weighing 0. 33 g that contained 0.0291 g (0.0978 mmol) of zinc nitrate hexahydrate was added to 1 g of the partially deboronated silicate obtained in Synthetic Example 1. The zinc ions were allowed to impregnate the silicate by the incipient wetness method.

After impregnated with the solution, the powder was sufficiently agitated and was calcined in air at 120°C for 3 hours and at 500°C for 4 hours. Thus, zinc-supported zeolite was prepared.

(Synthetic Example 6) Preparation of platinum- and zinc-supported silicate (catalyst 3)

Platinum- and zinc-supported silicate (catalyst 3) was prepared in the same manner as in Synthetic Example 3, except that the zinc-supported silicate obtained in Synthetic Example 2 was replaced by the zinc-supported zeolite obtained in Synthetic Example 5.

The (supported) amount of platinum and the (supported) amount of zinc in the catalyst 3 were determined by ICP-AES to be 0.32% by mass and 0.64% by mass, respectively.

(Synthetic Example 7) Preparation of platinum- and zinc-supported MFI silicalite (catalyst 4)

Zinc-supported MFI silicalite was prepared in the same manner as in Synthetic Example 2, except that the partially deboronated silicate (zeolite) was replaced by MFI silicalite (silica/alumina ratio = 200000) that originally contained no boron atoms.

Platinum- and zinc-supported MFI silicalite (catalyst 4) was prepared in the same manner as in Synthetic Example 3, except that the zinc-supported silicate obtained in Synthetic Example 2 was replaced by the zinc-supported MFI silicalite obtained above.

The (supported) amount of platinum and the (supported) amount of zinc in the MFI silicalite catalyst 4 were determined by ICP-AES to be 0.32% by mass and 1.78% by mass, respectively.

(Synthetic Example 8) Preparation of platinum- and zinc-supported borosilicate (catalyst 5)

Zinc-supported borosilicate was prepared in the same manner as in Synthetic Example 5, except that the partially deboronated silicate (zeolite) was replaced by borosilicate without the boron removal, namely, MFI borosilicate containing 3200 ppm of boron atoms which had been prepared by the method described later in Synthetic Example 11.

Platinum- and zinc-supported borosilicate (catalyst 5) was prepared in the same manner as in Synthetic Example 6, except that the zinc-supported zeolite obtained in Synthetic Example 5 was replaced by the zinc-supported borosilicate obtained above.

The (supported) amount of platinum and the (supported) amount of zinc in the borosilicate catalyst 5 were determined by ICP-AES to be 0.32% by mass and 0.64% by mass, respectively.

(Synthetic Example 9) Preparation of platinum- and zinc-supported silicate (catalyst 6)

Partially deboronated silicate (zeolite) was prepared in the same manner as in Synthetic Example 1, except that 9.23 g of the MFI borosilicate (silica/alumina ratio = 200000) containing 3200 ppm of boron atoms was replaced by MFI borosilicate (silica/alumina ratio = 3000) containing 3800 ppm of boron atoms which had been purchased from N. E. CHEMCAT CORPORATION.

The amount of the boron atoms in the silicate powder was determined to be 170 ppm by ICP-AES (residual rate of the boron atoms: approximately 4%).

Zinc-supported silicate was prepared in the same manner as in Synthetic Example 5, except that the above silicate was used. Platinum- and zinc-supported silicate (catalyst 6) was obtained in the same manner as in Synthetic Example 3, except that the above zinc-supported silicate was used.

The (supported) amount of platinum and the (supported) amount of zinc in the silicate catalyst 6 were determined by ICP-AES to be 0.32% by mass and 0.64% by mass, respectively.

(Synthetic Example 10) Preparation of platinum- and zinc-supported silicate (catalyst 7)

Silicate powder was obtained in the same manner as in Synthetic Example 1, except that the amount of the 3 N aqueous nitric acid solution was changed from 900 ml to 180 ml, and that the filtered residue was not subjected to the second cycle of the treatments, namely, the treatments were terminated when the residue was washed with 300 ml of distilled water. The amount of the boron atoms in the silicate powder was determined to be 1300 ppm by ICP-AES (residual rate of the boron atoms: approximately 40%).

Zinc-supported silicate was prepared in the same manner as in Synthetic Example 2, except that the above silicate was used. Platinum- and zinc-supported silicate (catalyst 7) was obtained in the same manner as in Synthetic Example 3, except that the above zinc-supported borosilicate was used.

The (supported) amount of platinum and the (supported) amount of zinc in the catalyst 7 were determined by ICP-AES to be 0.32% by mass and 1.78% by mass, respectively.

(Synthetic Example 11) Synthesis of MFI borosilicate

A 1.2 L volume, Teflon-lined stainless steel autoclave was charged with 371 g of a 22.5% by mass aqueous tetrapropylammonium hydroxide solution, 267 g of distilled water and 23.5 g of boric acid, followed by stirring at room temperature for 10 minutes. Further, 111 g of fumed silica (AEROSIL® 380) was added. The mixture was stirred for a day at room temperature. The resultant slurry liquid was slowly heated under stirring. The reaction was performed at 170°C for 6 days. The product was washed, filtered, dried at 120°C for 4 hours, and calcined at 540°C for 6 hours to give powder. The obtained powder was added to 3 L of a 1 mol/L aqueous ammonium nitrate solution. After the mixture was stirred at 80°C for 3 hours, the liquid was filtered and the residue was washed. The residue was again treated with the aqueous ammonium nitrate solution, filtered and washed. The solid residue was dried at 120°C for 4 hours and calcined at 540°C for 6 hours to give white powder. The white powder was identified to have an MFI crystal structure by X-ray powder diffractometry. The MFI borosilicate was analyzed by ICP-AES and ICP-MS, resulting in a boron atom content of 3200 ppm and a silica/alumina ratio of 200000.

The properties of the catalysts 1 to 7 prepared in Synthetic Examples 1 to 11 are described in TABLE 1.

[Table 1]

TABLE 1 List of catalysts

| | | | Zeolites | B | Residual B rate | Si/Al2 | Pt | Zn |
|---|---|---|---|---|---|---|---|---|
| | | | | ppm | % | Molar ratio | wt% | wt% |
| Cat. | 1 | Syn. Ex. 3 | Deboronated silicate (zeolite) | 260 | 8 | 200000 | 0.32 | 1.78 |
| Cat. | 2 | Syn. Ex. 4 | Borosilicate (without deboronation) | 3200 | 100 | 200000 | 0.32 | 1.78 |
| Cat. | 3 | Syn. Ex. 6 | Deboronated silicate (zeolite) | 260 | 8 | 200000 | 0.32 | 0.64 |
| Cat. | 4 | Syn. Ex 7 | Silicalite | - | - | 200000 | 0.32 | 1.78 |
| Cat. | 5 | Syn. Ex. 8 | Borosilicate (without deboronation) | 3200 | 100 | 200000 | 0.32 | 0.65 |
| Cat. | 6 | Syn. Ex. 9 | Deboronated silicate (zeolite) | 170 | 4 | 3000 | 0.32 | 0.64 |
| Cat. | 7 | Syn. Ex. 10 | Deboronated silicate (zeolite) | 1300 | 40 | 200000 | 0.32 | 1.78 |

(Measurement of platinum surface area in synthesized catalysts)

The platinum surface areas of the catalyst 3 and the borosilicate catalyst 5 were calculated from the amount of carbon monoxide adsorbed on platinum.

The measurement procedures were as follows. Each catalyst in an amount of 0.1 g was placed into a U-shaped glass sample tube, and the tube was set in a carbon monoxide adsorption apparatus. At room temperature, the gas phase of the sample tube was purged with helium gas and thereafter with hydrogen gas. The catalyst was then pretreated by increasing the temperature to 600°C in 30 minutes and holding the temperature at 600 °C for 2 hours under a stream of hydrogen, thereby reducing the catalyst.

Thereafter, in a stream of helium gas, the temperature was decreased to 50°C, and helium gas containing 10% by volume of carbon monoxide was introduced 5 times (as 5 pulses) to the catalyst layer. The carbon monoxide in the gas that had passed the catalyst layer was quantitatively determined with a thermal conductivity detector.

The amount of carbon monoxide adsorbed to the catalyst layer, and the platinum surface area per 1 g of platinum were calculated from the difference between the amount of the introduced carbon monoxide and the amount of the detected carbon monoxide. As a result, the platinum surface areas per 1 g of platinum in the catalyst 3 (Synthetic Example 6) and the catalyst 5 (Synthetic Example 8) were 66.6 $m^2$ and 27.5 $m^2$, respectively. This result showed that the at least partially deboronated silicate (zeolite) as the catalyst support permitted platinum particles to be deposited thereon with greatly improved dispersibility compared with the borosilicate without the boron removal. Accordingly, the lattice defects formed by the boron atom removal according to the invention were demonstrated to improve the dispersibility of the supported metals. Similar effects are expected as long as defects are formed in the zeolite crystal by other than the boron atom removal. In such cases, the use of the borosilicate as the precursor is not compulsory.

[Example 1]

0.25 g of the catalyst 1 from Synthetic Example 3 was loaded into a tubular reactor (a SUS tube) 1/2 inch in diameter and 300 mm in total length which had an alumina inner tube 6 mm in inner diameter. Further, quartz sand was loaded to fill the air gaps.

The reactor was connected with a flow reactor. After nitrogen was passed through the reactor, hydrogen was fed (20 ml/min) and the temperature was raised to 600°C with an electric furnace. After the temperature of the reactor reached 600°C, the catalyst 1 was reduced with hydrogen for 2 hours.

After the reduction for 2 hours, n-butane was fed to the reactor at a rate of 2 g/h that was controlled with a mass flow controller, thereby initiating the reaction (WHSV = 8 $h^{-1}$). The reaction was performed at 0.15 MPa. After the start of the reaction, the products after the lapse of the predetermined time shown in TABLE 2 were analyzed by on-line gas chromatography. The line from the reactor exit to the gas chromatographs was temperature-controlled at 220°C.

The quantitative determination of the products was conducted by an absolute calibration method using a gas chromatograph fitted with a hydrogen flame ionization detector and a gas chromatograph equipped with a thermal conductivity detector. The results of the product analysis are described in TABLE 2.

The butane conversion, the butene selectivity and the butadiene selectivity were defined as follows. The concentration was on the mass basis. The same applies to other Examples and Comparative Examples that will be presented later.

Butane conversion = ((n-butane concentration in materials + isobutane concentration in materials) - (n-butane concentration in products + isobutane concentration in products))/(n-butane concentration in materials + isobutane concentration in materials)

Butene selectivity = (1-butene concentration in products + 2-butene concentration in products + isobutene concentration in products)/((n-butane concentration in materials + isobutane concentration in materials) - (n-butane concentration in products + isobutane concentration in products - hydrogen concentration in products))

Butadiene selectivity = butadiene concentration in products/((n-butane concentration in materials + isobutane concentration in materials) - (n-butane concentration in products + isobutane concentration in products - hydrogen concentration in products))

[Table 2]

| TABLE 2 Results of dehydrogenation of n-butane (Ex. 1) | | | | | | | | | | | | | |
|---|---|---|---|---|---|---|---|---|---|---|---|---|---|
| Reaction corditions | Time on stream (h) | 1 | 15 | 27 | 39 | 51 | 63 | 75 | 87 | 99 | 111 | 123 | 135 |
| Products | hydrogen | 2.4 | 2.2 | 2.2 | 2.0 | 2.0 | 1.8 | 1.7 | 1.3 | 1.0 | 0.7 | 0.4 | 0.2 |
| (mass %) | C1-C3 compounds | 11.3 | 9.9 | 7.7 | 7.7 | 7.6 | 7.3 | 7.2 | 7.2 | 5.9 | 5.9 | 5.5 | 5.6 |
| | n-butane + isobutane | 29.6 | 29.6 | 31.6 | 32.3 | 39.0 | 37.6 | 42.7 | 53.2 | 65.1 | 74.6 | 82.5 | 88.5 |
| | 1-butene + 2-butene + isobutene | 50.1 | 53.9 | 54.4 | 54.0 | 52.6 | 49.7 | 44.9 | 35.3 | 25.9 | 17.4 | 10.9 | 5.4 |
| | butadiene | 1.1 | 2.7 | 3.3 | 3.2 | 3.1 | 3.0 | 2.9 | 2.5 | 1.8 | 1.2 | 0.6 | 0.2 |
| | C5 and higher compounds | 1.8 | 0.9 | 0.6 | 0.6 | 0.5 | 0.5 | 0.4 | 0.4 | 0.2 | 0.2 | 0.1 | 0.1 |
| | benzene + toluene + xylene | 3.8 | 0.7 | 0.3 | 0.2 | 0.2 | 0.1 | 0.1 | 0.1 | 0.0 | 0.0 | 0.0 | 0.0 |
| Results | Butane conversion (%) | 70.4 | 70.4 | 68.4 | 67.7 | 66.0 | 62.4 | 57.3 | 46.8 | 34.9 | 25.4 | 17 5 | 11.5 |
| | Butene selectivity (%) | 73.7 | 79.4 | 82.4 | 82.6 | 82.5 | 82.4 | 81.2 | 78.1 | 77.1 | 70.9 | 64.2 | 48.4 |
| | Butadiene selectivity (%) | 1.6 | 4.0 | 4.9 | 4.9 | 4.9 | 5.0 | 5.2 | 5.6 | 5.3 | 5.0 | 3.7 | 2.1 |
| Reaction conditions reaction temperature 600°C, pressure 0.15 MPa, butane feed rate 2 g/h | | | | | | | | | | | | | |
| | Cat. 1: 0.32 wt% Pt 0.64 wt% Zn/deboronated MFI zeolite 0.25 g | | | | | | | | | | | | |

[Example 2]

Dehydrogenation reaction was carried out in the same manner as in Example 1, except that the catalyst 1 from Synthetic Example 3 was replaced by the catalyst 3 from Synthetic Example 6. The results of the product analysis are described in TABLE 3.

[Table 3]

| TABLE 3 Results of dehydrogenation of n-butane (Ex. 2) | | | | | | | | | | | | | |
|---|---|---|---|---|---|---|---|---|---|---|---|---|---|
| Reaction conditions Time on stream (h) | | 1 | 13 | 25 | 37 | 50 | 62 | 74 | 86 | 98 | 110 | 122 | 134 |
| Products (mass %) | hydrogen | 2.1 | 2.2 | 2.2 | 2.2 | 2.2 | 2.1 | 2.1 | 2.0 | 1.9 | 1.5 | 1.2 | 0.8 |
| | C1-C3 compounds | 16.4 | 7.2 | 6.5 | 5.8 | 5.4 | 5.2 | 5.1 | 4.9 | 4.8 | 4.6 | 4.4 | 4.5 |
| | n-butane + isobutane | 26.7 | 31.6 | 7.3 | 33.4 | 34.7 | 35.5 | 37.2 | 41.1 | 45.9 | 57.8 | 65.5 | 75.8 |
| | 1-butene + 2-butene + isobutene | 49.2 | 54.2 | 54.2 | 54.0 | 53.1 | 52.6 | 51.1 | 47.7 | 43.4 | 32.7 | 26.0 | 16.8 |
| | butadiene | 3.4 | 4.3 | 4.3 | 4.3 | 4.3 | 4.3 | 4.2 | 4.0 | 3.8 | 3.3 | 2.7 | 2.0 |
| | C5 and higher compounds | 0.4 | 0.3 | 0.2 | 0.2 | 0.2 | 0.2 | 0.2 | 0.2 | 0.2 | 0.1 | 0.1 | 0.1 |
| | benzene + toluene + xylene | 1.0 | 0.2 | 0.1 | 0.1 | 0.1 | 0.1 | 0.1 | 0.1 | 0.0 | 0.0 | 0.0 | 0.0 |
| Results | Butane conversion (%) | 73.3 | 68.4 | 67.6 | 66.6 | 65.3 | 64.5 | 62.8 | 58.9 | 54.1 | 42.2 | 34.5 | 24.2 |
| | Butene selectivity (%) | 69.0 | 81.8 | 83.0 | 83.8 | 84.2 | 84.3 | 84.2 | 83.9 | 83.1 | 80.2 | 78.0 | 72.0 |
| | Butadiene selectivity (%) | 4.8 | 6.5 | 6.6 | 6.6 | 6.8 | 6.8 | 6.9 | 7.1 | 7.3 | 8.2 | 8.2 | 8.5 |
| Reaction conditions: reaction temperature 600°C, pressure 0.15 MPa, butane feed rate 2 g/h | | | | | | | | | | | | | |
| | Cat 3: 0.32 wt% Pt 0.64 wt% Zn/deboronated MFI zeolite 0.25 g | | | | | | | | | | | | |

[Example 3]

Dehydrogenation reaction was carried out in the same manner as in Example 2, except that a n-butane/water mixture gas was fed to the reactor instead of n-butane and that the reaction pressure was 0.1 MPa. The feed rate of n-butane was 2 g/h (= about 0.034 mol/h) and the feed rate of water was 1.2 g/h (= about 0.067 mol/h) (i.e., water/n-butane molar ratio = about 2). The results of the product analysis are described in TABLE 4.

[Table 4]

| TABLE 4 Results of dehydrogenation of n-butane (Ex. 3) | | | | | | | | | | | | | | | | | | | | |
|---|---|---|---|---|---|---|---|---|---|---|---|---|---|---|---|---|---|---|---|---|
| Reaction conditions | Time on stream (h) | 1 | 13 | 22 | 37 | 52 | 73 | 103 | 124 | 148 | 184 | 221 | 260 | 299 | 348 | 382 | 424 | 466 | 496 | 520 |
| Products | hydrogen | 2.8 | 3.3 | 3.0 | 3.5 | 3.6 | 3.5 | 3.4 | 3 5 | 3.1 | 3.5 | 3.4 | 3.3 | 3.3 | 3.4 | 3.3 | 3.1 | 2.8 | 2.5 | 2.0 |
| (mass %) | C1-C3 compounds | 4.8 | 1.8 | 2.0 | 1.8 | 1.9 | 1.8 | 1.8 | 1.6 | 1.7 | 1.5 | 1.2 | 1.4 | 1.5 | 1.5 | 1.4 | 1.5 | 0.8 | 0.8 | 0.6 |
| | n-butane + isobutane | 38.2 | 27.4 | 23.1 | 20.5 | 18.7 | 18.5 | 19.1 | 19.4 | 19.3 | 20.4 | 23.1 | 22.2 | 21.3 | 22.9 | 24.1 | 27.0 | 43.3 | 41.3 | 53.4 |
| | 1-butene + 2-butene + isobutene | 46.7 | 56.2 | 59.6 | 62.7 | 63.6 | 64.6 | 62.5 | 63.8 | 64.0 | 62.5 | 60.3 | 61.0 | 61.9 | 59. | 58.8 | 55.6 | 41.3 | 44.7 | 34.5 |
| | butadiene | 7.2 | 11.2 | 12.3 | 11.5 | 12.2 | 11.6 | 13.2 | 11.7 | 11.9 | 12.1 | 12.0 | 12.2 | 12.0 | 12.8 | 12.4 | 12.7 | 11.8 | 10.7 | 9.5 |
| | C5 and higher compounds | 0.1 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| | benzene + toluene + xylene | 0.2 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| Results | Butane conversion (%) | 61.8 | 72.6 | 76.9 | 79.5 | 81.3 | 81.5 | 80.9 | 80.6 | 80.7 | 79.6 | 76.9 | 77.8 | 78.7 | 77.1 | 75.9 | 73.0 | 56.7 | 58.7 | 46.6 |
| | Butene selectivity (%) | 79.1 | 81.1 | 80.6 | 82.5 | 81.9 | 82.8 | 80.6 | 82.7 | 82.4 | 82.0 | 82.0 | 81.8 | 82.1 | 80.6 | 81.0 | 79.6 | 76.6 | 79.5 | 77.2 |
| | Butadiene selectivity (%) | 12.3 | 16.2 | 16.6 | 15.1 | 15.7 | 14.8 | 17.1 | 15.2 | 15.4 | 15.9 | 16.4 | 16.3 | 15.9 | 17.3 | 17.1 | 18.1 | 21.9 | 19.1 | 21.4 |
| Reaction conditions reaction temperature 600°C, pressure 0.1 MPa, butane feed rate 2 g/h, water feed rate 1.2 g/h | | | | | | | | | | | | | | | | | | | | |

EP 2 604 590 A1

(continued)

| TABLE 4 Results of dehydrogenation of n-butane (Ex. 3) | | | | | | | | | | | | | | | | | | | | |
|---|---|---|---|---|---|---|---|---|---|---|---|---|---|---|---|---|---|---|---|---|
| Reaction conditions | Time on stream (h) | 1 | 13 | 22 | 37 | 52 | 73 | 103 | 124 | 148 | 184 | 221 | 260 | 299 | 348 | 382 | 424 | 466 | 496 | 520 |
| | Cat. 3: 0.32 wt% Pt 0.64 wt% Zn/deboronated MFX zeolite 0.25 g | | | | | | | | | | | | | | | | | | | |

[Example 4]

Dehydrogenation reaction was carried out in the same manner as in Example 3, except that the catalyst 3 from Synthetic Example 6 was replaced by the catalyst 6 from Synthetic Example 9. The results of the product analysis are described in TABLE 5.
[Table 5]

TABLE 5 Results of dehydrogenation of n-butane (Ex. 4)

| | Time on stream (h) | 1 | 13 | 22 | 56 | 96 | 132 | 168 | 204 | 237 | 255 | 273 | 291 | 309 | 327 | 368 | 404 | 425 | 443 | 461 |
|---|---|---|---|---|---|---|---|---|---|---|---|---|---|---|---|---|---|---|---|---|
| Reaction conditions | | | | | | | | | | | | | | | | | | | | |
| Products (mass%) | hydrogen | 4.4 | 2.9 | 3.1 | 3.1 | 3.5 | 3.2 | 3.8 | 3.5 | 3.3 | 3.4 | 3.3 | 3.2 | 3.3 | 3.1 | 3.0 | 3.0 | 2.7 | 2.6 | 2.5 |
| | C1-C3 compounds | 11.4 | 2.3 | 2.3 | 2.2 | 2.5 | 2.4 | 3.0 | 3.0 | 3.0 | 3.0 | 3.3 | 2.8 | 2.3 | 1.8 | 1.3 | 1.0 | 0.8 | 0.7 | 0.7 |
| | n-butane + isobutane | 21.5 | 24.9 | 20.0 | 17.3 | 14.9 | 15.9 | 14.0 | 14.5 | 15.0 | 15.8 | 15.3 | 17.0 | 18.6 | 20.5 | 24.5 | 29.4 | 31.7 | 34.3 | 36.1 |
| | 1-butene + 2-butene + isobutene | 55.0 | 62.1 | 64.4 | 67.0 | 67.1 | 67.4 | 66.0 | 66.9 | 66.7 | 65.8 | 65.9 | 65.1 | 63.6 | 62.6 | 60.1 | 56.0 | 55.0 | 53.2 | 51.8 |
| | butadiene | 4.7 | 7.2 | 10.0 | 10.3 | 12.0 | 11.1 | 12.4 | 12.2 | 11.8 | 12.0 | 12.2 | 11.9 | 12.3 | 11.9 | 11.1 | 10.6 | 9.7 | 9.1 | 8.8 |
| | C5 and higher compounds | 0.2 | 0.2 | 0.1 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| | benzene + toluene + xylene | 2.3 | 0.3 | 0.1 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 | 0.0 |
| Results | Buane conversion (%) | 78.5 | 75.1 | 80.0 | 82.7 | 85.1 | 84.1 | 86.0 | 85.5 | 85.0 | 84.2 | 84.7 | 83.0 | 81.4 | 74.5 | 75.5 | 70.6 | 68.3 | 65.7 | 63.9 |
| | Butene selectivity (%) | 74.2 | 86.0 | 83.7 | 84.2 | 82.2 | 83.3 | 81.2 | 81.5 | 81.8 | 81.4 | 81.0 | 81.6 | 81.4 | 82.0 | 82.9 | 82.8 | 83.8 | 84.3 | 84.4 |
| | Butadiene selectivity (%) | 6.0 | 9.6 | 12.5 | 12.4 | 14.1 | 13.1 | 14.4 | 14.2 | 13.9 | 14.2 | 14.4 | 14.3 | 15.1 | 15.0 | 14.6 | 15.0 | 14.3 | 13.9 | 13.8 |

| TABLE 5 Results of dehydrogenation of n-butane (Ex. 4) | | | | | | | | | | | | | | | | | | | | |
|---|---|---|---|---|---|---|---|---|---|---|---|---|---|---|---|---|---|---|---|---|
| Reaction conditions | Time on stream (h) | 1 | 13 | 22 | 56 | 96 | 132 | 168 | 204 | 237 | 255 | 273 | 291 | 309 | 327 | 368 | 404 | 425 | 443 | 461 |
| Reaction conditions: reaction temperature 600°C, pressure 0.1 MPa, butane feed rate 2 g/h, water feed rate 1.2 g/h | | | | | | | | | | | | | | | | | | | | |
| | Cat. 6: 0.32 wt% Pt 0.64 wt% Zn/deboronated MFI zeolite 0.25 g | | | | | | | | | | | | | | | | | | | | |

**EP 2 604 590 A1**

[Example 5]

Dehydrogenation reaction was carried out in the same manner as in Example 3, except that a n-butene/water mixture gas was fed to the reactor instead of the n-butane/water mixture gas. The feed rate of n-butene was 2 g/h (= about 0.036 mol/h) and the feed rate of water was 4.8 g/h (= about 0.27 mol/h) (i.e., water/n-butene molar ratio = about 7.5). The results of the product analysis are described in TABLE 6.

In Example 5, carbon monoxide and carbon dioxide in the products were analyzed during a reaction time of 25 hours. The weight-based selectivity was 1.7% for carbon monoxide and 0.8% for carbon dioxide. This result suggested that the coke which was a cause for catalyst deactivation was oxidized by water into carbon monoxide and carbon dioxide, and consequently the catalytic life was extended.

[Table 6]

| TABLE 6 Results of dehydrogenation of n-butene (Ex 5) | | | | | | | | | | | | |
|---|---|---|---|---|---|---|---|---|---|---|---|---|
| Reaction conditions | Time on stream (h) | 1 | 13 | 25 | 37 | 49 | 61 | 73 | 85 | 97 | 109 | 115 |
| Products | hydrogen | 2.1 | 1.5 | 1.6 | 1.5 | 1.5 | 1.7 | 1.5 | 1.6 | 1.2 | 1.3 | 1.4 |
| (mass %) | C1-C3 compounds | 7.6 | 6.8 | 5.8 | 5.2 | 4.4 | 4.1 | 3.5 | 3.4 | 1.9 | 2.4 | 1.7 |
| | n-butane + isobutane | 2.9 | 3.2 | 3.0 | 2.5 | 2.3 | 1.9 | 1.6 | 1.3 | 1.6 | 1.0 | 1.0 |
| | 1-butene + 2-butene + isobutene | 60.8 | 57.0 | 57.4 | 57.2 | 58.3 | 57.1 | 57.4 | 56.9 | 62.9 | 59.5 | 63.2 |
| | butadiene | 26.5 | 31.3 | 32.2 | 33.5 | 33.4 | 35.2 | 36.0 | 36.8 | 32.3 | 35.7 | 32.6 |
| | C5 and higher compounds | 0.0 | 0.1 | 0.1 | 0.1 | 0.1 | 0.1 | 0.1 | 0.1 | 0.1 | 0.1 | 0.1 |
| | benzene + toluene + xylene | 0.1 | 0.0 | 0.0 | 0.0 | 0.0 | 0.0 | 0.0 | 0.0 | 0.0 | 0.0 | 0.0 |
| Results | Butene conversion (%) | 39.2 | 43.0 | 42.6 | 42.8 | 41.7 | 42.9 | 42.6 | 43.1 | 37.1 | 40.5 | 36.8 |
| | Butadiene selectivity (%) | 71.4 | 75.7 | 78.3 | 81.3 | 83.2 | 85.4 | 87.5 | 88.6 | 90.0 | 91.1 | 92.2 |
| Reaction conditions: reaction temperature 600°C, pressure 0.1 MPa, butene feed rate 2 g/h, water feed rate 4.8 g/h | | | | | | | | | | | | |
| | Cat. 3- 0.32 wt% Pt 0.64 wt% Zn/deboronated MFI zeolite 0.25 g | | | | | | | | | | | |

[Example 6]

Dehydrogenation reaction was carried out in the same manner as in Example 1, except that the catalyst 1 was replaced by the catalyst 7. The results of the product analysis are described in TABLE 7.

[Table 7]

| TABLE 7 Results of dehydrogenation of n-butane (Ex. 6) | | | | | | | | |
|---|---|---|---|---|---|---|---|---|
| Reaction conditions | Time on stream (h) | 1 | 13 | 25 | 49 | 67 | 85 | 97 |
| Products | hydrogen | 2.6 | 2.3 | 2.2 | 2.0 | 1.6 | 1.3 | 0.6 |
| (mass %) | C1-C3 compounds | 13.6 | 9.4 | 8.6 | 6.7 | 6.1 | 5.4 | 3.6 |
| | n-butane + isobutane | 27.2 | 34.2 | 34.6 | 40.2 | 52.2 | 60.6 | 80.5 |
| | 1-butene + 2-butene + isobutene | 49.6 | 48.6 | 49.9 | 46.7 | 36.5 | 29.7 | 13.8 |
| | butadiene | 2.5 | 3.0 | 4.1 | 4.0 | 3.4 | 3.0 | 1.5 |
| | C5 and higher compounds | 0.5 | 0.3 | 0.2 | 0.2 | 0.1 | 0.1 | 0.0 |
| | benzene + toluene + xylene | 3.3 | 1.8 | 0.3 | 0.2 | 0.1 | 0.1 | 0.0 |
| Results | Butane conversion (%) | 72.8 | 65.8 | 65.4 | 59.8 | 47.8 | 39.4 | 19.5 |
| | Butene selectivity (%) | 70.7 | 77.2 | 79.3 | 81.1 | 79.2 | 78.1 | 73.1 |
| | Butadiene selectivity (%) | 3.5 | 4.9 | 6.4 | 7.0 | 7.5 | 7.8 | 7.8 |
| Reaction conditions: reaction temperature 600°C, pressure 0.15 MPa, butane feed rate 2 g/h | | | | | | | | |
| | Cat. 7: 0.32 wt% Pt 1.78 wt% Zn/deboronated MFI zeolite 0.25 g | | | | | | | |

[Example 7]

Dehydrogenation reaction was carried out in the same manner as in Example 3, except that the reaction temperature was 450°C. Analyzing the products showed that the n-butene yield was 26% which was the equilibrium value. The yield did not decrease after the reaction was carried out for at least 120 hours. The selectivity of n-butene was 98%.

[Example 8]

Dehydrogenation reaction was carried out in the same manner as in Example 2, except that propane/water mixture gas was fed to the reactor instead of n-butane and that the reaction pressure was 0.1 MPa. The feed rate of propane was 2 g/h (= about 0.045 mol/h). The results of the product analysis are described in TABLE 8.

[Table 8]

| TABLE 8 Results of dehydrogenation of propane (Ex. 8) | | | | |
|---|---|---|---|---|
| Reaction conditions | Time on stream (h) | 1 | 7 | 13 |
| Products | hydrogen | 2.1 | 2.0 | 2.1 |
| (mass %) | C1-C2 compounds | 1.7 | 1.7 | 1.5 |
| | propane | 55.5 | 55.0 | 54.9 |
| | propylene | 39.9 | 40.6 | 41.0 |
| | C4 compound | 0.3 | 0.4 | 0.3 |
| | C5 and higher compounds | 0.1 | 0.1 | 0.1 |
| | benzene + toluene + xylene | 0.4 | 0.2 | 0.1 |

(continued)

| TABLE 8 Results of dehydrogenation of propane (Ex. 8) | | | | |
|---|---|---|---|---|
| Reaction conditions | Time on stream (h) | 1 | 7 | 13 |
| Results | Propane conversion (%) | 44.5 | 45.0 | 45.1 |
| | Propylene selectivity (%) | 94.1 | 94.7 | 95.5 |
| Reaction conditions: reaction temperature 600°C, propane feed rate 2 g/h | | | | |
| 0.32 wt% Pt 1.78 wt% Zn/deboronated MFI zeolite 0.25 g | | | | |

[Comparative Example 1]

Dehydrogenation reaction was carried out in the same manner as in Example 1, except that the catalyst 1 from Synthetic Example 3 was replaced by the borosilicate catalyst 2 from Synthetic Example 4. The results of the product analysis are described in TABLE 9.

[Table 9]

| TABLE 9 Results of dehydrogenation of n-butane (Comp Ex. 1) | | | | | | | | | | | |
|---|---|---|---|---|---|---|---|---|---|---|---|
| Reaction conditions | Time on stream (h) | 1 | 13 | 25 | 37 | 49 | 61 | 73 | 85 | 97 | 109 |
| Products | hydrogen | 2.3 | 2.2 | 2.1 | 1.8 | 1.5 | 1.0 | 0.6 | 0.1 | 0.1 | 0.0 |
| (mass %) | C1-C3 compounds | 6.8 | 7.5 | 6.7 | 6.5 | 6.0 | 6.2 | 5.9 | 6.4 | 6.3 | 6.5 |
| | n-butane + isobutane | 38.1 | 34.9 | 39.3 | 46.7 | 58.0 | 68.2 | 80.9 | 91.5 | 92.7 | 92.6 |
| | 1-butene + 2-butene + isobutene | 48.6 | 50.9 | 47.9 | 41.4 | 31.4 | 22.1 | 11.4 | 2.0 | 0.9 | 0.8 |
| | butadiene | 2.8 | 4.1 | 3.7 | 3.5 | 3.0 | 2.4 | 1.1 | 0.1 | 0.0 | 0.0 |
| | C5 and higher compounds | 0.2 | 0.2 | 0.1 | 0.1 | 0.1 | 0.1 | 0.0 | 0.0 | 0.0 | 0.0 |
| | benzene + toluene + xylene | 1.1 | 0.3 | 0.1 | 0.1 | 0.0 | 0.0 | 0.0 | 0.0 | 0.0 | 0.0 |
| Results | Butane conversion (%) | 61.9 | 65.1 | 60.7 | 53.3 | 42.0 | 31.8 | 19.1 | 8.5 | 7.3 | 7.4 |
| | Butene selectivity (%) | 81.3 | 81.0 | 81.7 | 80.5 | 77.5 | 72.0 | 62.1 | 23.7 | 13.2 | 11.7 |
| | Butadiene selectivity (%) | 4.7 | 6.6 | 6.4 | 6.7 | 7.5 | 7.8 | 6.1 | 0.9 | 0.4 | 0.4 |
| Reaction conditions: reaction temperature 600°C, pressure 0.15 MPa, butane feed rate 2 g/h | | | | | | | | | | | |
| Cat. 2: 0.32 wt% Pt 1.78 wt% Zn/MFI borosilicate 0.25 g | | | | | | | | | | | |

[Comparative Example 2]

Dehydrogenation reaction was carried out in the same manner as in Example 1, except that the catalyst 1 from Synthetic Example 3 was replaced by the MFI silicalite catalyst 4 from Synthetic Example 7. The results of the product analysis are described in TABLE 10.

[Table 10]

| TABLE 10 Results of dehydrogenation of n-butane (Comp. Ex 2) | | | | | | | | | | | | |
|---|---|---|---|---|---|---|---|---|---|---|---|---|
| Reaction conditions | Time on stream (h) | 1 | 13 | 25 | 37 | 49 | 61 | 73 | 85 | 97 | 109 | 115 |
| Products | hydrogen | 2.4 | 2.2 | 2.3 | 2.0 | 1.9 | 1.4 | 1.0 | 0.3 | 0.1 | 0.1 | 0.1 |
| (mass %) | C1-C3 compounds | 6.2 | 6.7 | 6.7 | 6.1 | 6.1 | 5.6 | 5.6 | 5.3 | 5.9 | 5.7 | 5.7 |
| | n-butane + isobutane | 38.6 | 36.4 | 36.3 | 40.6 | 46.6 | 58.3 | 70.6 | 86.7 | 92.5 | 93.3 | 93.3 |
| | 1-butene + 2-butene + isobutene | 49.4 | 50.8 | 50.5 | 47.0 | 41.4 | 31.3 | 20.1 | 7.0 | 1.4 | 0.9 | 0.9 |
| | butadiene | 1.4 | 3.6 | 3.9 | 3.9 | 3.8 | 3.3 | 2.5 | 0.6 | 0.1 | 0.0 | 0.0 |
| | C5 and higher compounds | 0.3 | 0.1 | 0.1 | 0.1 | 0.1 | 0.1 | 0.1 | 0.0 | 0.0 | 0.0 | 0.0 |
| | benzene + toluene + xylene | 1.7 | 0.3 | 0.2 | 0.1 | 0.1 | 0.1 | 0.0 | 0.0 | 0.0 | 0.0 | 0.0 |
| Results | Butane conversion (%) | 61.4 | 63.6 | 63.7 | 59.4 | 53.4 | 41.7 | 29.4 | 13.3 | 7.5 | 6.7 | 6.7 |
| | Butene selectivity (%) | 83.3 | 82.7 | 82.1 | 82.1 | 80.2 | 77.7 | 70.9 | 54.4 | 19.3 | 14.6 | 13.9 |
| | Butadiene selectivity (%) | 2.5 | 6.1 | 6.6 | 7.0 | 7.7 | 8.6 | 9.3 | 5.2 | 0.9 | 0.6 | 0.5 |
| Reaction conditions: reaction temperature 600°C, pressure 0.15 MPa, butane feed rate 2 g/h | | | | | | | | | | | | |
| | Cat 4: 0.32 wt% Pt 1.78 wt% Zn/silicalite 0.25 g | | | | | | | | | | | |

[Comparative Example 3]

Dehydrogenation reaction was carried out in the same manner as in Example 3, except that the catalyst 3 from Synthetic Example 6 was replaced by the borosilicate catalyst 5 from Synthetic Example 8. The results of the product analysis are described in TABLE 11.

[Table 11]

| TABLE 11 Results of dehydrogenation of n-butane (Comp Ex 3) | | | | | | | | | | | | |
|---|---|---|---|---|---|---|---|---|---|---|---|---|
| Reaction conditions | Time on stream (h) | 1 | 10 | 16 | 25 | 28 | 34 | 37 | 43 | 46 | 58 | 64 |
| Products (mass %) | hydrogen | 2.7 | 2.3 | 2.2 | 2.1 | 2.1 | 2.1 | 2.1 | 2.0 | 2.0 | 1.9 | 1.8 |
| | C1-C3 compounds | 2.0 | 1.4 | 1.2 | 1.3 | 1.2 | 1.1 | 1.1 | 1.0 | 1.0 | 1.1 | 0.9 |
| | n-butane + isobutane | 28.2 | 35.7 | 38.5 | 38.7 | 37.8 | 37.7 | 38.0 | 40.4 | 42.0 | 46.5 | 48.2 |
| | 1-butene + 2-butene + isobutene | 57.5 | 51.5 | 49.3 | 49.4 | 49.9 | 50.5 | 50.1 | 48.1 | 46.0 | 41.6 | 40.6 |
| | butadiene | 9.4 | 8.8 | 8.6 | 8.4 | 8.7 | 8.2 | 8.4 | 8.3 | 8.7 | 8.6 | 8.1 |
| | C5 and higher compounds | 0.2 | 0.3 | 0.3 | 0.2 | 0.4 | 0.3 | 0.3 | 0.3 | 0.3 | 0.3 | 0.3 |
| | benzene + toluene + xylene | 0.0 | 0.0 | 0.0 | 0.0 | 0.0 | 0.0 | 0.0 | 0.0 | 0.0 | 0.0 | 0.0 |
| Results | Butane conversion (%) | 71.8 | 64.3 | 61.5 | 61.3 | 62.2 | 62.3 | 62.0 | 59.6 | 58.0 | 53.5 | 51.8 |
| | Butene selectivity (%) | 83.1 | 83.1 | 83.0 | 83.3 | 83.0 | 84.0 | 83.7 | 83.4 | 82.2 | 80.7 | 81.4 |
| | Butadiene selectivity (%) | 13.5 | 14.2 | 14.5 | 14.1 | 14.4 | 13.7 | 14.0 | 14.4 | 15.5 | 16.7 | 16.3 |
| Reaction conditions: reaction temperature 600°C, pressure 0.10 MPa, butane feed rate 2 g/h, water feed rate 1.2 g/h | | | | | | | | | | | | |
| | Cat. 5. 0.32 wt% Pt 0.64 wt% Zn/MFI borosilicate 0.25 g | | | | | | | | | | | |

The results in TABLES 2 to 11 show that the catalysts A according to the present invention can catalyze the dehydrogenation of hydrocarbons for a longer time (longer catalytic life) than the borosilicate catalysts used without the boron removal in Comparative Examples 1 and 3 or the (originally boron-free) MFI silicalite catalyst used in Comparative Example 2, regardless of the presence or absence of water in the reaction system. In particular, the catalyst 5 that had not been subjected to the boron removal did not substantially extend the catalytic life even by the addition of water to the reaction system. In contrast, the catalysts A of the invention were demonstrated to drastically extend the catalytic life by the presence of water in the reaction system.

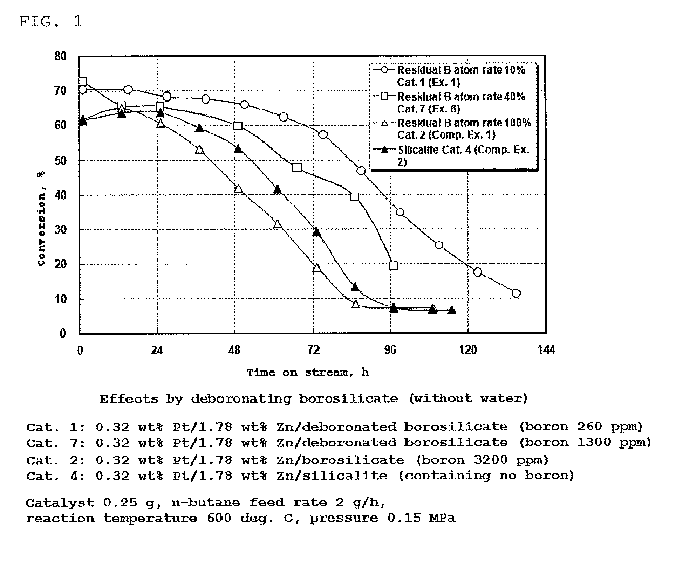

For reference, the difference of the catalytic life of the catalysts used in Examples 1 and 6 and Comparative Examples 1 and 2 is visually illustrated in FIG. 1.

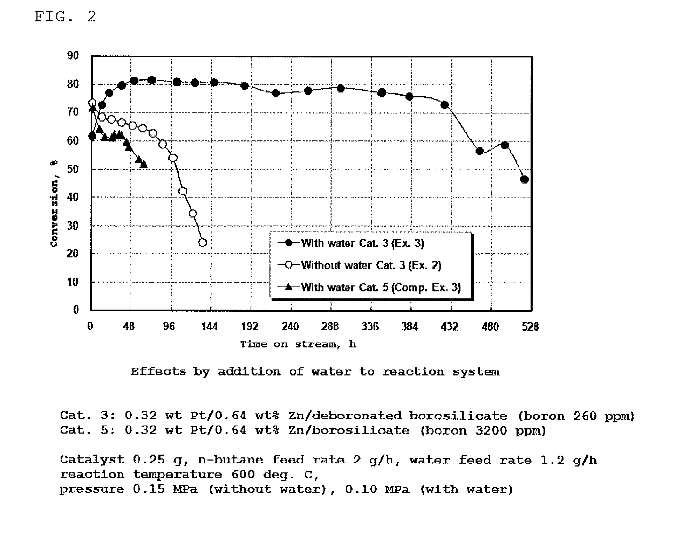

Further, FIG. 2 visually illustrates the difference of the catalytic life of the catalysts used in Example 2 (water was absent from the reaction system), Example 3 (water was present in the reaction system) and Comparative Example 3 (water was present in the reaction system).

INDUSTRIAL APPLICABILITY

According to the inventive process for producing unsaturated hydrocarbons described hereinabove, unsaturated hydrocarbons, preferably olefins and/or dienes that are useful as industrial materials, can be manufactured using the catalyst having a longer catalytic life than known dehydrogenation catalysts.

In particular, the production process of the invention can produce:

propylene which is useful as a material in the synthesis of, for example, acrylonitrile, polypropylene, ethylene/propylene rubber, propylene oxide, acetone, isopropyl alcohol and octanol;

1-butene and 2-butene which are useful as materials for sec-butyl alcohol, butadiene and propylene;

isobutene which is a highly valuable substance as a material for polyisobutylene, methacrolein, methyl methacrylate, methyl tert-butyl ether, ethyl tert-butyl ether, dibutylhydroxytoluene and dibutylhydroxyanisole;

butadiene, producible from 1-butene or 2-butene as an intermediate, which is used as a feedstock for synthetic rubbers such as SBR and NBR, ABS resins and nylon 66; and

styrene, producible by the dehydrogenation of ethylbenzene, which is useful as a feedstock for polystyrene that is a general resin.

According to the process for producing unsaturated hydrocarbons of the invention, the catalyst regeneration frequency is reduced and the catalytic life is further drastically extended by allowing water to be present in the reaction system. Accordingly, the invention increases the freedom in designing the dehydrogenation process and facility and is expected to realize industrially advantageous production of unsaturated hydrocarbons.

Further, the catalyst A according to the invention is free of highly toxic chromium. Thus, the production process of the invention is also excellent in terms of the safety aspect.

**Claims**

1.  A process for producing unsaturated hydrocarbons, comprising a step of dehydrogenating a hydrocarbon into a corresponding unsaturated hydrocarbon by contacting the hydrocarbon with a catalyst A that is obtained by supporting zinc and a Group VIIIA metal on a silicate obtained by removing at least part of the boron atoms from a borosilicate.

2.  The process for producing unsaturated hydrocarbons according to claim 1, wherein in the catalyst A, the hydrocarbon is an unsaturated hydrocarbon.

3.  The process for producing unsaturated hydrocarbons according to claim 1 or 2, wherein in the catalyst A, the residual rate of the boron atoms in the silicate is not more than 80% relative to all the boron atoms in the borosilicate.

4.  The process for producing unsaturated hydrocarbons according to any one of claims 1 to 3, wherein the amount of the zinc in the catalyst A is 0.01 to 15% by mass.

5.  The process for producing unsaturated hydrocarbons according to any one of claims 1 to 4, wherein the amount of the Group VIIIA metal in the catalyst A is 0.01 to 5% by mass.

6.  The process for producing unsaturated hydrocarbons according to any one of claims 1 to 5, wherein the Group VIIIA metal is platinum.

7.  The process for producing unsaturated hydrocarbons according to any one of claims 1 to 6, wherein the borosilicate is a MFI zeolite.

8.  The process for producing unsaturated hydrocarbons according to any one of claims 1 to 7, wherein the hydrocarbon has 2 to 20 carbon atoms.

9.  The process for producing unsaturated hydrocarbons according to any one of claims 1 to 8, wherein the unsaturated hydrocarbon is an olefin and/or a diene.

10. The process for producing unsaturated hydrocarbons according to any one of claims 1 to 9, wherein the hydrocarbon is at least one selected from the group consisting of propane, n-butane, isobutane and isopentane.

11. The process for producing unsaturated hydrocarbons according to claim 1, wherein the hydrocarbon is at least one selected from the group consisting of n-butene and ethylbenzene.

12. The process for producing unsaturated hydrocarbons according to any one of claims 1 to 11, wherein the contacting of the hydrocarbon and the catalyst A is performed at a reaction temperature of 300 to 800°C.

13. The process for producing unsaturated hydrocarbons according to any one of claims 1 to 12, wherein the contacting of the hydrocarbon and the catalyst A is performed at a reaction pressure of 0.01 to 1 MPa.

14. The process for producing unsaturated hydrocarbons according to any one of claims 1 to 13, wherein the contacting of the hydrocarbon and the catalyst A is performed in the presence of water.

15. The process for producing unsaturated hydrocarbons according to claim 14, wherein the usage amount of water is 0.05 to 20 molar times relative to the hydrocarbon.

16. A catalyst A which is obtained by supporting zinc and a Group VIIIA metal on a silicate obtained by removing at least part of the boron atoms from a borosilicate, and which catalyzes the dehydrogenation of a hydrocarbon into a corresponding unsaturated hydrocarbon.

FIG. 1

Effects by deboronating borosilicate (without water)

Cat. 1: 0.32 wt% Pt/1.78 wt% Zn/deboronated borosilicate (boron 260 ppm)
Cat. 7: 0.32 wt% Pt/1.78 wt% Zn/deboronated borosilicate (boron 1300 ppm)
Cat. 2: 0.32 wt% Pt/1.78 wt% Zn/borosilicate (boron 3200 ppm)
Cat. 4: 0.32 wt% Pt/1.78 wt% Zn/silicalite (containing no boron)

Catalyst 0.25 g, n-butane feed rate 2 g/h,
reaction temperature 600 deg. C, pressure 0.15 MPa

FIG. 2

Effects by addition of water to reaction system

Cat. 3: 0.32 wt Pt/0.64 wt% Zn/deboronated borosilicate (boron 260 ppm)
Cat. 5: 0.32 wt Pt/0.64 wt% Zn/borosilicate (boron 3200 ppm)

Catalyst 0.25 g, n-butane feed rate 2 g/h, water feed rate 1.2 g/h
reaction temperature 600 deg. C,
pressure 0.15 MPa (without water), 0.10 MPa (with water)

<div align="center">

**INTERNATIONAL SEARCH REPORT**

</div>

| | International application No. |
|---|---|
| | PCT/JP2011/068098 |

**A. CLASSIFICATION OF SUBJECT MATTER**
*C07C5/333*(2006.01)i, *B01J29/86*(2006.01)i, *C07C11/08*(2006.01)i, *C07C11/09*(2006.01)i, *C07C11/167*(2006.01)i, *C07B61/00*(2006.01)n

According to International Patent Classification (IPC) or to both national classification and IPC

**B. FIELDS SEARCHED**

Minimum documentation searched (classification system followed by classification symbols)
C07C5/333, B01J29/86, C07C11/08, C07C11/09, C07C11/167, C07B61/00

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | US 6103103 A (BP Amoco Corp.), 15 August 2000 (15.08.2000), claims 1 to 10; table 1 & EP 768995 A1 & WO 1996/001239 A1 | 1-16 |
| A | JP 8-196909 A (INTEVEP S.A.), 06 August 1996 (06.08.1996), claims 1 to 37 (Family: none) | 1-16 |
| A | US 4433190 A (Standard Oil Co. (Indiana)), 21 February 1984 (21.02.1984), claims 1 to 28 (Family: none) | 1-16 |

| ☒ Further documents are listed in the continuation of Box C. | ☐ See patent family annex. |
|---|---|

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search 31 October, 2011 (31.10.11) | Date of mailing of the international search report 08 November, 2011 (08.11.11) |
|---|---|
| Name and mailing address of the ISA/ Japanese Patent Office | Authorized officer |
| Facsimile No. | Telephone No. |

Form PCT/ISA/210 (second sheet) (July 2009)

**EP 2 604 590 A1**

<table>
<tr><td colspan="2" style="text-align:center">**INTERNATIONAL SEARCH REPORT**</td><td>International application No.<br>PCT/JP2011/068098</td></tr>
</table>

C (Continuation). DOCUMENTS CONSIDERED TO BE RELEVANT

| Category* | Citation of document, with indication, where appropriate, of the relevant passages | Relevant to claim No. |
|---|---|---|
| A | JP 4-504439 A (Chevron Research and Technology Co.),<br>06 August 1992 (06.08.1992),<br>claims 1 to 32<br>& US 5114565 A  & EP 465606 A1<br>& WO 1991/011501 A1 | 1-16 |
| A | US 2002/0004624 A1 (BP Amoco Corp.),<br>10 January 2002 (10.01.2002),<br>claims 1 to 16<br>& EP 1251961 A1  & WO 2001/052984 A1 | 1-16 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0278439 A
- US 4433190 A
- US 6197717 B
- US 6555724 B
- US 20020004624 A
- US 5114565 A
- US 5324702 A

**Non-patent literature cited in the description**

- *Catal. Today,* 1999, vol. 51, 223
- *Canadian Chemical News,* 1984, vol. 10, 1924
- *Kinetics and Catalysis,* 2001, vol. 42, 72
- *Petrochemie,* 1995, vol. 111, 171
- Handbook of Heterogeneous Catalysis. VCH, 1997, vol. 5, 2151